(19) Europäisches Patentamt

European Patent Office

Office européen des brevets

(11) Publication number: **0 315 944 B1**

(12) **EUROPEAN PATENT SPECIFICATION**

(45) Date of publication of patent specification: **27.04.94**

(51) Int. Cl.5: **C12P 1/00**, C12M 1/36, //C12P17/18,C12N15/00

(21) Application number: **88118567.2**

(22) Date of filing: **08.11.88**

(54) Controlled growth rate fermentation.

(30) Priority: **09.11.87 US 118702**

(43) Date of publication of application:
**17.05.89 Bulletin 89/20**

(45) Publication of the grant of the patent:
**27.04.94 Bulletin 94/17**

(84) Designated Contracting States:
**DE GB**

(56) References cited:
**EP-A- 0 078 500**

**BIOTECH. PROCEEDINGS OF THE INTERNATIONAL CONFERENCE ON THE COMMERCIAL APPLICATIONS OF BIOTECHNOLOGY, London, 4th - 6th May 1983, pages 295-306, Online Conf., Ltd, Northwood, GB; T. SUMITANI et al.: "Automatic control of fermentor using microcomputer"**

(73) Proprietor: **GENENCOR INTERNATIONAL, INC.**
**180 Kimball Way**
**South San Francisco, CA 94080(US)**

(72) Inventor: **Lee, Sooyoung Stanford c/o Eastman Kodak Company**
**Patent Department**
**343 State Street**
**Rochester New York 14650(US)**
Inventor: **Mohler, Robert Douglas c/o Eastman Kodak Company**
**Patent Department**
**343 State Street**
**Rochester New York 14650(US)**

(74) Representative: **Brandes, Jürgen, Dr. rer. nat. et al**
**Wuesthoff & Wuesthoff**
**Patent- und Rechtsanwälte**
**Schweigerstrasse 2**
**D-81541 München (DE)**

**Description**

In large scale fermentation processes, it is common to operate the fermentation in a batch mode. In such a process, the initial culture is added to a fermentor which already contains all of the nutrients to achieve the desired final cell mass.

In many batch fermentations, the high concentrations of the initial starting materials are detrimental to the optimum growth of the microorganism. As a result, the so called fed batch process is common. In such a process, the nutrients are added as the fermentation progresses so as to improve the cell mass that is produced or the growth rate or other parameters.

The advent of recombinant DNA technology has focused additional attention on fermentation processes. The exogenous DNA is usually carried in a microorganism that must be grown in a fermentor in order to produce the desired product. During the growth phase or thereafter, the exogenous DNA is expressed and the desired product produced.

Usually, the host microorganism is an Escherichia coli or a Saccharomyces cerevisiae although certain Bacillus, Pseudomonas and Streptomyces species are beginning to be used. Unfortunately, these microorganisms, when grown at the maximum rate attainable, produce undesirable byproducts. These byproducts not only result in the diversion of starting material and energy, but ultimately limit the amount of cells that can be produced and also the rate at which they are produced.

In E. coli, for example, it is known that unrestrained growth produces acetic acid which, we have found, prematurely ends the logarithmic growth phase of the microorganism. It would be desirable to prolong the logarithmic growth phase of the microorganism particularly where the desired product is being produced during this phase.

Controlling the process by monitoring the acetic acid produced is not practical. First, analytical methods for on-line measurement of acetate are not well developed. More importantly, however, we have found that by the time acetate is detected, the physiology of the microorganism has already changed and less than optimum results are achieved if control is attempted at this time.

In Zabriskie and Acuri, Factors influencing productivity of fermentations employing recombinant microorganisms, Enzyme Microb. Technol., vol 8, pg. 706-717, (1986) it is suggested that improvements in this type of fermentation can be achieved if the growth rate is restricted to less than the maximum value. To achieve this, carbon source is fed at a constant rate that is below the rate that will produce maximum growth rate. In such a strategy, the growth rate will always be changing even though it will always be below the maximum value attainable at any particular point in time.

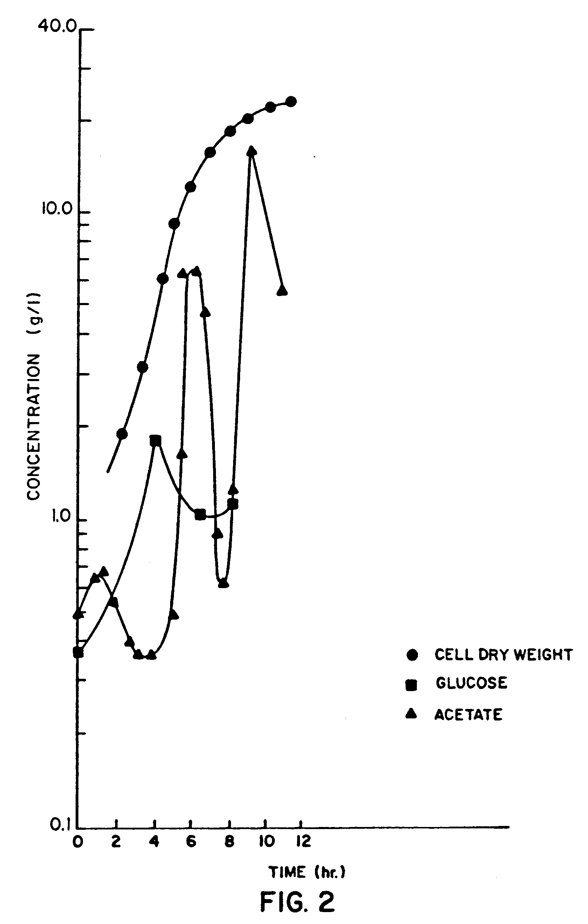

As the title suggests, Allen and Luli, A Gradient-Feed Process for Obtaining High Cell Densities for Recombinant E. Coli.,(1985), describe a process wherein the feed rate in a fed batch fermentation is varied during the course of the fermentation. Periodic off-line measurements of the glucose and the cell density were used to update an algorithm that was used to calculate the feed rate. Pertinent features of Figure 4 of the paper are reproduced as Figure 2 herein. In this typical fermentation according to Allen and Luli, three different growth rates were observed. In addition, the acetate production, while lower than in an uncontrolled fermentation, was significant. The excess glucose was significant during much of the fermentation. Finally, the process requires constant operator attention and is difficult to run reproducibly. It is clear that while the process of Allen and Luli is an improvement over the art, still further improvements in the fed batch process for microorganisms are desirable.

Thus, the problem to be solved by the present invention is to provide a fed batch process that substantially reduces or eliminates the production of a growth inhibitory byproduct for as long as possible thereby prolonging the logarithmic growth phase.

In accordance with the present invention there is provided a fed batch process for the fermentation of a microorganism wherein a carbon source is added during the process, said process including the steps of:

1) continuously measuring the amount of carbon dioxide produced during the fermentation,

2) calculating the specific growth rate of the microorganism from the carbon dioxide produced, and

3) maintaining a substantially constant specific growth rate that is lower than the growth rate that results in an inhibitory accumulation of a byproduct by varying the feed rate of said carbon source.

The fermentation of E. coli is illustrative of the present invention. If, during the fermentation of E. coli, the growth rate is substantially constant and is always below the level that will produce acetate, the logarithmic growth phase continues until factors other than acetate limit the growth. This results in a much longer logarithmic growth phase. Longer logarithmic growth phase results in higher cell densities and improved product production, particularly where the product is produced during the growth phase. In addition, since an undesirable byproduct is not produced, the yield of the process is improved. No carbon is diverted to acetate production. Also because acetate is not produced, there are fewer problems with

2

spent medium disposal. Finally, control of the process using carbon dioxide measurement is easier than using off-line glucose and cell density measurement. Control based on carbon dioxide can be easily done on-line and automatically by computer.

Figure 1 is a plot of pertinent data from a process according to the present invention. Growth of E. coli, amount of acetate in the fermentation broth and glucose in the fermentation broth is plotted against time.

Figure 2 is a plot that is similar to Fig. 1 except that the process is according to the Allen and Luli reference cited above and is therefore not illustrative of the present invention.

Figure 3 is a schematic representation of carbon dioxide based feed back control used in the invention.

In the description which follows, details will be discussed for the fermentation of E. coli where the growth inhibitory byproduct is acetate. It will be understood however, that the invention is directed to a process using any microorganism, including, for example, species from the genera Saccharomyces, e. g. cerevisiae, Bacillus Pseudomonas and Streptomyces. Also, while the emphasis in the present description will be on the inhibitory action of acetate, it will be understood that the invention can be used to reduce the effect of other inhibitory materials such as other organic acids.

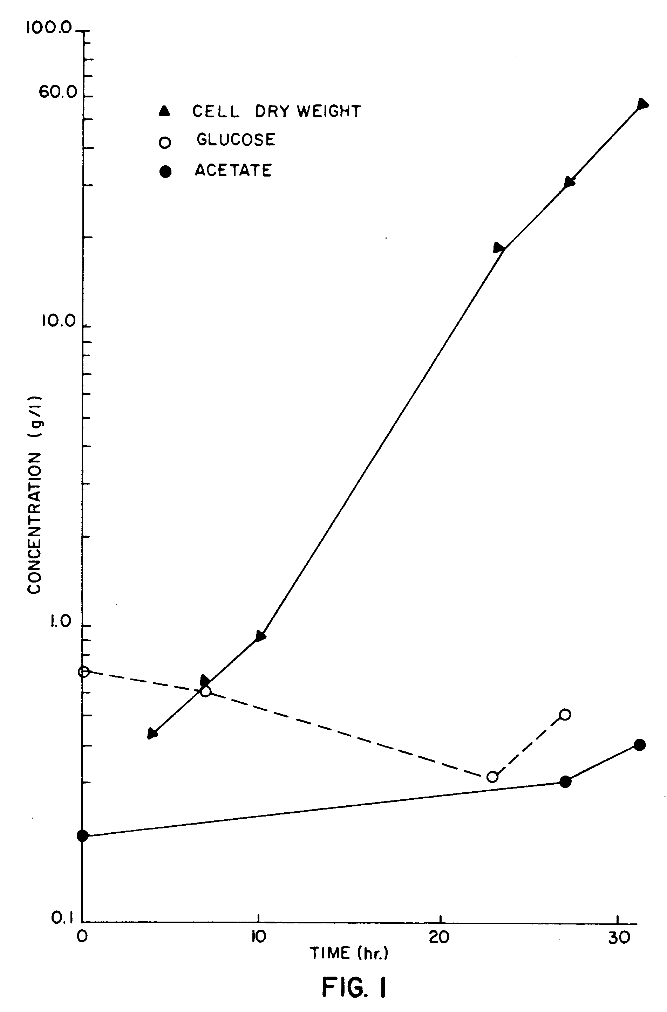

Figure 1 is a plot of the results of a typical process according to the present invention. The details regarding the fermentation that produced these results are described in Example 1. It should be noted that, after a brief initial high rate growth period (not plotted), the growth rate remained constant. During the process, samples of the medium were taken and analyzed for acetate and glucose. Both of these materials remained at low levels during the process.

Figure 1 should be contrasted with Figure 2 which is reproduced from the data in the Allen and Luli paper described above. Their process also involved the growth of E. coli and changes in the feed rate of the carbon source. However, feed rate was changed based on off-line measurements of glucose and cell density. No attempt was made to achieve a constant growth rate. Rather, the growth rate was reduced periodically in an attempt to reduce the acetate produced. As a result of this form of control, three growth rates were observed and noticeable amounts of acetate were produced. In addition, the medium contained, at various times, excess glucose.

According to the present invention, the specific growth rate is held at a substantially constant rate by varying the feed rate. If the specific growth rate increases above the desired amount, the feed rate is reduced. Conversely, if the growth rate is below the desired amount, the feed rate is increased.

The specific growth rate is defined as the present rate of growth divided by the total amount of growth that has taken place up to the present time. The specific growth rate therefore has the units of 1/time. For the purposes of the invention, it is assumed that the the rate of carbon dioxide produced is directly proportional to the rate of growth and, similarly, that the total amount of carbon dioxide produced is directly proportional to the total amount of growth. Thus, the specific growth rate can be constantly monitored using carbon dioxide data.

Carbon dioxide produced in a fermentor is commonly measured and therefore this measurement is well within the skill of those in the art. One method is to use an on-line mass spectrometer.

The growth rate is maintained at a substantially constant rate that is lower than the rate that produces an inhibitory accumulation of a growth inhibitory byproduct. The exact growth rate that is used in the process is dependent on the microorganism that is being grown. Even within a particular species, e. g. E. coli, the exact desired rate will vary from strain to strain and will also depend on the medium that is used. It therefore must be determined experimentally. Methods for determining the growth rate at which inhibitory products are produced are known in the art. In a typical method, the microorganism is grown in a continuous reactor under steady state conditions. This is known in this art as a chemostat. Chemostats require long periods of time to come to equilibrium and therefore are not useful for commercial purposes.

The chemostat growth of a strain of recombinant E. coli, illustrating the growth rate which begins to produce acetate, has been published and illustrates a method for determining the desired growth rate in the fed batch process of the invention. Reference is made to Fiescho and Ritch, Chem. Eng. Commun., 45 229-240, 1986. Threshold growth rates in other microorganisms which might produce other growth inhibitory byproducts, can be determined in a similar manner.

Alternatively, the present controlled growth rate process can be used to determine the exact growth rate for subsequent fermentations. For example, if a selected growth rate in a particular fermentation produces excess inhibitory byproduct, a subsequent fermentation can be carried out at a slightly lower growth rate.

It should be noted that a growth rate that is slightly higher than the growth rate that produces no inhibitory byproduct might be used in some circumstances. What is important is that the inhibitory byproduct be produced at such a rate that it does not accumulate to inhibitory levels during the course of the fermentation. In most instances, it will be desirable to select a growth-rate such that no inhibitory byproduct is produced.

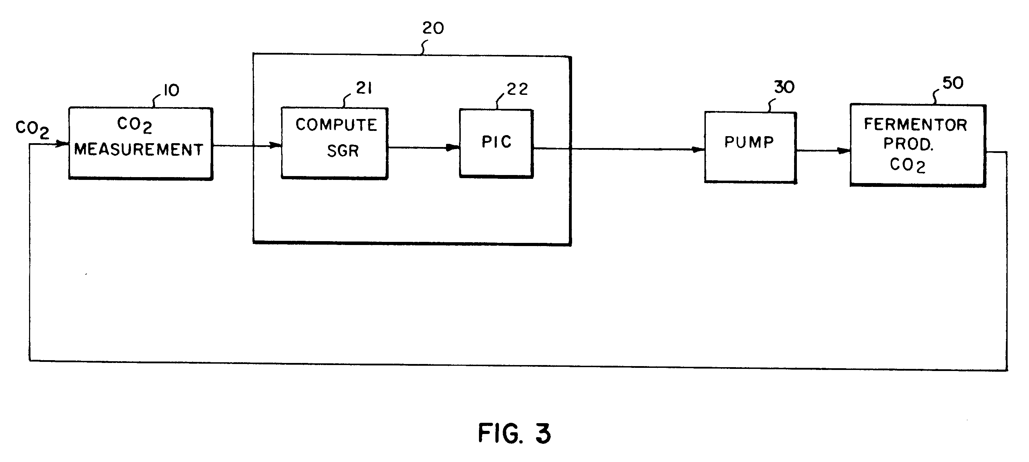

In Fig. 3 there is shown a block diagram of the equipment that is useful in carrying out the control that is part of the present invention. There is shown a carbon dioxide measurement device 10, which can be, for example, a mass spectrometer. Output from this device is to computer 20 which includes means for computing the specific growth rate (SGR) 21 and logic means for controlling the process 22. Logic means 22 can be a proportional integral controller (PIC) which controls feed pump 40. Carbon dioxide produced in the fermentor 50 is then sampled by the carbon dioxide measurement device 10 completing the control circuit.

In the batch fermentation according to the invention, the medium that is used to grow the microorganism is not critical. Any combination of ingredients that have been found to be useful for the growth of the particular microorganism can be used. It is desirable that the proportions of the ingredients in the feed be such that the resultant medium that is in the fermentor be substantially carbon source limited so that control based on carbon dioxide is effective. Optimization of the medium is well within the skill of those in the art. The initial amount of carbon source in the fermentor at start up should be low so that the growth rate falls to the desired control range early in the process.

Other parameters known to be important in fermentations are controlled in conventional manners. For example, it is desirable to include pH control, dissolved oxygen control and temperature control.

The invention is particularly useful where a useful product is produced by the microorganism during the growth phase since the growth phase is extended according to the invention. Many products that are produced by exogenous DNA fall in this category. However, since higher ultimate cell densities are achieved, the invention is also useful where the desired product is produced during a later stationary phase or simply where it is desired to produce the microorganism in high cell densities.

In one embodiment, the invention is used with a microorganism that contains exogenous DNA for the production of biotin. Such a recombinant microorganism is E. coli BM4062 containing the plasmid pKa5 as described in PCT application 8,701,391 published 12 March 1987. Using the invention, biotin productivities of 2.2 mg/L-hr have been achieved in comparison with 1.0 mg/L-hr with a conventional fed batch process. Chemostat experiments showed that no acetate was produced by this microorganism if the specific growth rate was kept below about 0.1/hr at 30°C. Only a small amount of acetate was produced at a specific growth rate of 0.2/hr.

The following examples are presented for a further understanding of the invention:

Example 1:

A seed culture was prepared for inoculation into the production fed batch fermentor. This procedure was selected to minimize the production of acetate during this initial phase of the process. A flask which contained 500 mL of the tank medium described below was inoculated with 0.5 mL of a frozen vial which contained E. coli BM4062 containing plasmid pKa5. This flask was incubated while stirring at 200 rpm for 16 hours at 30°C.

The optical density of the resulting culture was obtained and a sample of the culture of sufficient size such that the product of the optical density and the mL of the sample was 140 (hereinafter mLOD) was used to inoculate a second flask containing another 500 mL of the tank medium. This again was incubated with stirring for 16 hrs at 30°C.

A sample containing 1960 mLOD was then used to inoculate a 14 L fed batch fermentor which contained 5 L of the tank medium.

The fed batch fermentor was equipped with the equipment described in relation to Fig. 3 above. The specific growth rate, as calculated by the carbon dioxide evolved, was controlled by a proportional integral controller which resided in a Hewlett Packard A-600 real-time process computer control system. The proportional integral algorithm was the digital equivalent of the following:

$$\text{Output} = K_c \epsilon + K_i \int_o^t \epsilon dt$$

The controller error $\epsilon$ was obtained by subtracting the calculated specific growth rate (from the carbon dioxide evolution), from the desired specific growth rate setpoint. The output from the controller was then used to change the rate of feed medium delivered to the fermentor. $K_c$ is the proportional gain (set to 10 in this example) and $K_i$ is the integral gain (set to 0.1 in this example). The fermentor was also equipped with conventional pH, dissolved oxygen and temperature controls.

Since the fermentor initially contains an amount of glucose, the growth rate did not fall into the controllable range for about 4 hours. After that period, the carbon dioxide control system previously described held the specific growth rate between 0.15 and .20/hr. When the optical density of the material in the fermentor reached 20, the temperature was raised to 37°C in order to increase the copy number of the plasmid in the microorganism. The acetate produced at this growth rate was minimal. (Maximum acetate concentration reached only 0.4 g/L after 34 hours.)

The compositions of the tank medium and the feed medium were as follows:

TABLE I

| Media Compositions | | |
|---|---|---|
| | Tank Media | Feed Media |
| glucose | 1.000 g/l | 440.000 g/l |
| $(NH_4)_2SO_4$ | 15.000 g/l | 30.000 g/l |
| $KH_2PO_4$ | 3.500 g/l | 3.500 g/l |
| $K_2HPO_4$ | 3.000 g/l | 3.000 g/l |
| methionine | 2.000 g/l | 2.000 g/l |
| $MgSO_4 \cdot 7H_2O$ | 1.233 g/l | 1.233 g/l |
| thiamine*HC1 | 0.0225 g/l | 0.0225 g/l |
| histidine | 1.500 g/l | 1.500 g/l |
| vitamin solution | 20.000 ml/l | 20.000 ml/l |
| trace metal | 10.000 ml/l | 10.000 ml/l |
| ampicillin | 0.100 g/l | 0.100 g/l |
| alanine | - | 0.500 g/l |
| $NaMoO_4$ | 0.045 g/l | 0.045 g/l |
| Vitamin Solution | | |
| Folic Acid | 0.005 g/l | |
| Pyridoxine $\cdot$ HCl | 0.175 g/l | |
| Riboflavin | 0.0525 g/l | |
| Pantothenic Acid Calcium Salt | 0.675 g/l | |
| Nicotinic Acid | 0.7625 g/l | |
| Trace Metal Solution | | |
| $FeCl_3 \cdot 6H_2O$ | 27.0 g/l | |
| $FnCl_2$ | 2.0 g/l | |
| $CoCl_2 \cdot 6H_2O$ | 2.0 g/l | |
| $CaCl_2 \cdot 2H_2O$ | 1.0 g/l | |
| $CuSO_4 \cdot 5H_2O$ | 1.9 g/l | |
| $H_3BO_3$ | 0.5 g/l | |
| $MnCl_2 \cdot 4H_2O$ | 1.6 g/l | |
| $Na_3$ citrate $\cdot 2H_2O$ | 73.5 g/l | |

The results are shown in Fig. 1. The logarithmic growth phase continued for 34 hours and the cell reached an optical density of 110. (In the Figure, cell dry weight is plotted. The relationship between cell dry weight and optical density is a factor of about 2. Thus, for example, when the cell dry weight is 60, the corresponding optical density of the corresponding fermentation medium is about 120.) Biotin production averaged 2.2 mg/ L-hr.

Example 2:

This is a comparative example.

Example 1 was repeated up through the point where the seed culture was placed in the fed batch fermentor. The fermentation was allowed to proceed. However, instead of controlling the growth rate, the rate of feed was just enough to supply a stoichiometric amount of glucose based on the carbon dioxide consumption.

The results were that the logarithmic growth phase continued for only about 20 hours, the cells reached an optical density of only about 60 and the biotin production average only about 1.0 g/L-hr over the 34 hour fermentation. Acetate production was significant during the first 15 hours of the process.

## Claims

1. A fed batch process for the fermentation of a microorganism wherein a carbon source is added during the process, said process including the steps of:
   1) continuously measuring the amount of carbon dioxide produced during the fermentation,
   2) calculating the specific growth rate of the microorganism from the carbon dioxide produced, and
   3) maintaining a substantially constant specific growth rate that is lower than the growth rate that results in an inhibitory accumulation of a byproduct by varying the feed rate of said carbon source.

2. A method according to claim 1 wherein said microorganism contains exogenous DNA.

3. A method according to claim 1 wherein said microorganism is a strain of E. coli.

4. A method according to claim 1 wherein said microorganism produces a desired product during the growth phase.

## Patentansprüche

1. Fad-Batch-Verfahren für die Fermentation eines Mikroorganismus, bei dem ein Kohlenstofflieferant während des Verfahrens zugegeben wird, wobei das Verfahren die folgenden Stufen einschließt:
   1) kontinuierliches Messen der Menge an Kohlendioxid, das während der Fermentation erzeugt wird,
   2) Berechnung der spezifischen Wachstumsgeschwindigkeit des Mikroorganismus aus dem erzeugten Kohlendioxid, und
   3) Aufrechterhaltung einer im wesentlichen spezifischen Wachstumsgeschwindigkeit, die geringer ist als die Wachstumsgeschwindigkeit, die bei einer hemmenden Akkumulation eines Nebenproduktes durch Veränderung der Einspeisgeschwindigkeit des Kohlenstofflieferanten resultiert.

2. Verfahren nach Anspruch 1, bei dem der Mikroorganismus exogene DNA enthält.

3. Verfahren nach Anspruch 1, bei dem der Mikroorganismus ein Stamm von E. coli ist.

4. Verfahren nach Anspruch 1, bei dem der Mikroorganismus ein gewünschtes Produkt während der Wachstumsphase erzeugt.

## Revendications

1. Procédé d'alimentation en discontinu pour la fermentation d'un micro-organisme, dans lequel une source de carbone est ajoutée durant le procédé, ledit procédé comprenant les étapes consistant à :
   1) mesurer continûment la quantité de dioxyde de carbone produite durant la fermentation,
   2) calculer la vitesse de croissance spécifique du micro-organisme d'après le dioxyde de carbone produit et
   3) maintenir une vitesse de croissance spécifique sensiblement constante, inférieure à la vitesse de croissance qui aboutit à une accumulation inhibitrice d'un sous-produit, par variation de la vitesse d'alimentation en ladite source de carbone.

2. Procédé selon la revendication 1, dans lequel ledit micro-organisme contient de l'ADN exogène.

3. Procédé selon la revendication 1, dans lequel ledit micro-organisme est une souche de E. coli.

4. Procédé selon la revendication 1, dans lequel ledit micro-organisme produit un produit désiré durant la phase de croissance.

FIG. 1

FIG. 2

FIG. 3

EP 0 315 944 B1